# EUROPÄISCHE PATENTSCHRIFT

(11) **EP 3 682 928 B1**
(45) Veröffentlichungstag und Bekanntmachung des Hinweises auf die Patenterteilung: **28.09.2022**
(21) Anmeldenummer: 20401001.1
(22) Anmeldetag: 15.01.2020
(51) Int. Cl.: A61M 16/00

(54) **BEATMUNGSGERÄT MIT ADAPTIVER STEUERUNG DER AUSATEMPHASE**
BREATHING APPARATUS WITH ADAPTIVE CONTROL OF EXHALATION PHASE
APPAREIL RESPIRATOIRE À COMMANDE ADAPTATIVE DE LA PHASE D'EXPIRATION

(30) Priorität: 21.01.2019 DE 102019101450
(43) Veröffentlichungstag der Anmeldung: 22.07.2020
(73) Patentinhaber: Löwenstein Medical Technology S.A., 2557 Luxembourg (LU)
(72) Erfinder: Kremeier, Peter, 76149 Karlsruhe (DE); Pulletz, Sven, 49090 Osnabrück (DE)
(74) Vertreter: Marx, Thomas

(56) Entgegenhaltungen:
- US-A1- 2006 011 195
- US-A1- 2009 114 222
- US-A1- 2009 205 663
- US-A1- 2015 290 408
- US-B2- 8 695 597

## Beschreibung

Die vorliegende Offenbarung bezieht sich im Allgemeinen auf die Bereitstellung einer Beatmung für Patienten, die eine Atmungsunterstützung benötigen.

Die bekannte CPAP-Therapie stellt ein oberes Druckniveau bereit, auf dem der Patient spontan atmen kann. Bei manchen Patienten reichert sich unter der CPAP-Therapie jedoch das Kohlendioxid der Ausatemluft an. Übliche Beatmungsformen begegnen diesem Problem mit einem geringen Ausatemdruck (EPAP), der abwechselnd mit einem hohen Druck für die Inspiration vorgegeben wird. Ein regelmäßiger Wechsel der Drücke für die Inspiration (IPAP) und die Exspiration (EPAP) ist jedoch nicht bei allen Patienten sinnvoll.

Es sind bereits verschiedene Ideen zur Verbesserung der Ausatmung bei einer CPAP-Therapie bekannt. So stellt die US 2015/0290408 A1 eine Methode vor, bei der ein konstantes hohes Druckniveau vorgesehen ist, welches auf ein niedriges Druckniveau abgesenkt werden kann. Die Umschaltung zwischen den Druckniveaus erfolgt dabei jeweils nach der erkannten Anstrengung des Patienten einzuatmen innerhalb eines vorgegebenen Zeitfensters.

Auch die US 8,695,597 B2 stellt einen Prozess vor, bei welchem eine längere Phase auf einem hohen Druckniveau beatmet wird, welches gelegentlich auf ein niedrigeres Druckniveau abgesenkt wird. Die Umschaltung von dem hohen auf das niedrige Druckniveau erfolgt dabei nach dem Erkennen einer Ausatemanstrengung innerhalb eines gewissen Zeitfensters.

Die US 2009/0205663 A1 offenbart zur Steuerung der Umschaltung zwischen einem hohen Druckniveau und niedrigen Druckniveau eine Abhängigkeit von dem Höchstwert des Ausatemflusses.

In der US 2009/0114222 A1 wird dazu beschrieben, dass das Umschalten auf Basis von EIT-Messungen möglich ist, wobei die reine Änderung in EIT-Werten zum Umschalten führen soll. Die US 2006/0011195 A1 beschreibt eine Methode, bei der folgend auf die Phase mit niedrigem Druckniveau eine Phase folgt, in welcher das Druckniveau über das Druckniveau der langen Beatmungsphase auf hohen Druckniveau angehoben wird. Die Umschaltung zwischen den einzelnen Phasen erfolgt beispielsweise zeitgesteuert und unabhängig vom Patienten oder auf die Atemphasen des Patienten synchronisiert.

Erfindungsgemäß wird daher von einem lang andauernden oberen Druckniveau kommend, der Druck für einen kurzen Zeitraum auf ein unteres Druckniveau abgesenkt. Durch dieses kurze Absenken ergibt sich ein größeres Ausatemvolumen; der PaCO2 fällt. Der erfindungsgemäße Modus erlaubt eine Vorgabe des Zeitraums für das lang andauernde obere Druckniveau und - in engen Grenzen - für das kurzzeitige untere Druckniveau, wobei das untere Niveau immer kürzer ist als das obere.

Die vorliegende Erfindung basiert auf der Erkenntnis, dass die Dauer, die Anzahl und das Ausmaß des unteren Druckniveaus stark abhängig ist vom Ausmaß der gewünschten Unterstützung der Ventilation und der Elimination von CO2. Die vorliegende Erfindung stellt sich daher zur Aufgabe, eine zumindest teilweise individuelle Einstellung von Ausmaß und Dauer des unteren Druckniveaus, mit einen geringen apparativen Aufwand, umzusetzen.

Die Erfindung ist in den beigefügten Ansprüchen definiert

Das erfindungsgemäße Beatmungsgerät weist eine Atemgasquelle und eine Steuereinheit auf die eingerichtet ist, Atemgas für einen ersten, längeren Zeitraum (Tinsp) auf einem oberen Druckniveau (Pinsp) bereitzustellen und Atemgas für einen zweiten, kürzeren Zeitraum Texsp) auf einem unteren Druckniveau (Pexsp) bereitzustellen, wobei das Beatmungsgerät Sensoren aufweist, die Messwerte des Atemgases (den Fluss und/oder Druck und/oder das Volumen des Atemgases) zumindest zeitweise erfassen wobei die Steuereinheit den ersten, längeren Zeitraum mit dem oberen Druckniveau für zumindest zwei spontane Atemphasen des Patienten (eine Inspiration und eine Exspiration) bereitstellt bevor der zweite, kürzere Zeitraum auf einem unteren Druckniveau vorgegeben wird, (um eine vertiefte Exspiration zu ermöglichen).

Das erfindungsgemäße Beatmungsgerät ist auch dadurch gekennzeichnet, dass das Beatmungsgerät Sensoren aufweist, die Messwerte des Atemgases (den Fluss und/oder Druck und/oder das Volumen des Atemgases) zumindest zeitweise erfassen wobei die Steuereinheit einen Übergang zwischen den Druckniveaus und/oder den zweiten Zeitraum zumindest teilweise in Abhängigkeit von den Messwerten steuert.

Das erfindungsgemäße Beatmungsgerät ist auch dadurch gekennzeichnet, dass die Steuereinheit den Übergang von dem unteren Druckniveau auf das obere Druckniveau in Abhängigkeit von den Messwerten für Fluss/Volumen steuert und der zweite Zeitraum insofern adaptiv ist.

Das erfindungsgemäße Beatmungsgerät ist auch dadurch gekennzeichnet, dass das Beatmungsgerät eine Spontanatmung eines Patienten auf dem oberen Druckniveau für zumindest zwei aufeinanderfolgende Atemphasen ermöglicht und dabei das Atemzugvolumen sensorisch erfasst und die Steuereinheit ein Basisniveau des Atemvolumens aus dem Messwert des Atemzugvolumens ermittelt.

Das erfindungsgemäße Beatmungsgerät weist eine Atemgasquelle und eine Steuereinheit auf, die eingerichtet ist Atemgas für einen ersten, längeren Zeitraum (Tinsp) auf einem oberen Druckniveau (Pinsp) bereitzustellen und Atemgas für einen zweiten, kürzeren Zeitraum (Texsp) auf einem unteren Druckniveau (Pexsp) bereitzustellen dadurch gekennzeichnet, dass das Beatmungsgerät Sensoren aufweist, die Messwerte des Atemgases (den Fluss und/oder Druck und/oder das Volumen des Atemgases) zumindest zeitweise erfassen wobei die Steuereinheit die Übergänge zwischen den Druckniveaus und den zweiten Zeitraum zumindest teilweise in Abhängigkeit von den Messwerten steuert.

Das erfindungsgemäße Beatmungsgerät ist auch eingerichtet, nur den zweiten Zeitraum zumindest teilweise in Abhängigkeit von den Messwerten zu steuern.

Das erfindungsgemäße Beatmungsgerät ist auch dadurch gekennzeichnet, dass die Steuereinheit den Übergang von dem unteren Druckniveau auf das obere Druckniveau in Abhängigkeit von den Messwerten für Fluss/Volumen steuert und der zweite Zeitraum insofern adaptiv ist.

Das erfindungsgemäße Beatmungsgerät ist auch dadurch gekennzeichnet, dass das Beatmungsgerät eine Spontanatmung eines Patienten auf dem oberen Druckniveau ermöglicht und dabei das Atemzugvolumen sensorisch erfasst und die Steuereinheit ein Basisniveau des Atemvolumens aus dem Messwert des Atemzugvolumens ermittelt, wobei die Steuereinheit während des zweiten Zeitraums das vom Patienten abgegebene Ausatemvolumen erfasst und die Zeitdauer des zweiten Zeitraums so lange andauert, bis das Ausatemvolumen den Wert des Basisniveaus im Wesentlichen erreicht hat.

Das erfindungsgemäße Beatmungsgerät ist auch dadurch gekennzeichnet, dass das Ausatemvolumen den Wert des Basisniveaus nicht unterschreitet.

Das erfindungsgemäße Beatmungsgerät ist alternativ auch dadurch gekennzeichnet, dass das Basisniveau (VT%) von der Steuereinheit aus dem Speicher als Vorgabewert entnommen wird. Das erfindungsgemäße Beatmungsgerät ist auch dadurch gekennzeichnet, dass das Beatmungsgerät in einer Lernphase den Inspirationsfluss beim Übergang von dem unteren Druckniveau auf das obere Druckniveau erfasst und den Exspirationsfluss beim Übergang von dem oberen Druckniveau auf das untere Druckniveau erfasst wobei das Beatmungsgerät nachfolgend eine Umschaltung von dem unteren Druckniveau auf das obere Druckniveau ansteuert, wenn der Fluss (auf dem unteren Druckniveau) einen Triggerwert erreicht hat, der einem Bruchteil der addierten Flüsse und entspricht.

Das Beatmungsgerät ist alternativ auch dadurch gekennzeichnet, dass der Triggerwert (28) + (29) / 2 entspricht.

Das erfindungsgemäße Beatmungsgerät ist alternativ auch dadurch gekennzeichnet, dass der Triggerwert sensorisch bestimmt wird und/oder durch einen Anwender vorgegeben wird (Total Flow %).

Das Beatmungsgerät weist beispielsweise eine Atemgasquelle auf, die eingerichtet ist, Atemgas für einen ersten Zeitraum auf einem oberen Druckniveau bereitzustellen und Atemgas für einen zweiten Zeitraum auf einem unteren Druckniveau bereitzustellen dadurch gekennzeichnet, dass das Beatmungsgerät den Inspirationsfluss beim Übergang von dem unteren Druckniveau auf das obere Druckniveau erfasst und das Beatmungsgerät den Exspirationsfluss beim Übergang von dem oberen Druckniveau auf das untere Druckniveau erfasst wobei das Beatmungsgerät eine Umschaltung von dem unteren Druckniveau auf das obere Druckniveau ansteuert, wenn der Fluss auf dem unteren Druckniveau einen Triggerwert erreicht hat, der einem Bruchteil der addierten Flüsse und entspricht.

Das Beatmungsgerät ist alternativ auch dadurch gekennzeichnet, dass die Zeitdauer des zweiten Zeitraums in dem Bereich 0,4 bis 1,8 sec, bevorzugt 0,5 - 3,0 sec liegt, in Abhängigkeit der Schwere der Oxygenierungsstörung.

Das Beatmungsgerät ist alternativ auch dadurch gekennzeichnet, dass die Anzahl der Phasen auf dem unteren Druckniveau 5 - 15 pro Min, bevorzugt 7 - 12 pro Min entspricht, abhängig vom Ausmaß der gewünschten Unterstützung der Ventilation und der Elimination von CO2. Das Beatmungsgerät ist alternativ auch dadurch gekennzeichnet, dass das Atemzeitverhältnis (Zeitraum Tinsp zu Zeitraum Texsp) größer 3 zu 1 ist, bevorzugt größer 4 zu 1 ist.

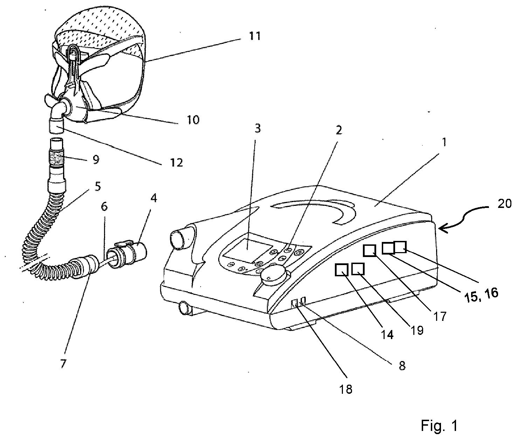

Fig. 1 zeigt den grundsätzlichen Aufbau eines Beatmungsgerätes (20). Im Bereich eines Gerätegehäuses (1) sind ein Bedienelement (2) und/oder ein Bedien- und Informationssystem (3) angeordnet. Über eine Kopplung (4) wird ein Verbindungsschlauch (5) angeschlossen. Entlang des Verbindungsschlauches (5) kann ein zusätzlicher Druckmessschlauch (6) verlaufen, der über einen Druckeingangsstutzen (7) mit dem Gerätegehäuse (1) verbindbar ist. Zur Ermöglichung einer Datenübertragung weist das Gerätegehäuse (1) zumindest eine Schnittstelle (8,18) auf. Ein Anfeuchter (21) oder ein Vernebler (22) kann zudem adaptiert werden. Das Beatmungsgerät weist eine Atemgasquelle (17) auf. Die Atemgasquelle kann als Elektromotor mit Lüfterrad ausgebildet sein oder als zumindest ein Ventil, welches den Atemgas-Strom oder Druck aus einer zentralen Atemgasleitung reguliert. Erfindungsgemäß sind auch Mischformen aus Elektromotor und Ventil denkbar.

Im Bereich einer dem Gerätegehäuse (1) abgewandten Ausdehnung des Verbindungsschlauches (5) ist beispielhaft ein Ausatmungselement (9) angeordnet. Ebenfalls kann ein Ausatemventil verwendet werden.

Das Beatmungsgerät (20) kann als Schlaftherapiegerät, als High-Flow-Gerät, als Anästhesiegerät, als klinisches- oder Heim- oder Notfall-Beatmungsgerät ausgebildet sein.

Fig. 1 zeigt darüber hinaus ein als Beatmungsmaske (10) ausgebildetes Patienten-Interface. Eine Fixierung im Bereich eines Kopfes eines Patienten kann über eine Kopfhaube (11) erfolgen. Im Bereich seiner dem Verbindungsschlauch (5) zugewandten Ausdehnung weist das Patienten-Interface (10) ein Kupplungselement (12) auf. Das Patienten-Interface kann beispielsweise auch als Tubus oder als ein Beatmungszugang ausgebildet sein.

Über die Schnittstelle (8,18) kann die Eingabe und / oder Ausgabe von Daten, wie beispielsweise Totraumvolumen, erfolgen. Die Schnittstellen können kabelgebunden, als Infrarot-Schnittstelle, als Bluetooth-Schnittstelle oder USB realisiert sein. Bevorzugt ist auch ein Kartenschacht vorgesehen. Die Schnittstelle (8) kann auch als LAN-Schnittstelle oder als sonstige Schnittstelle zur Anbindung an das Internet ausgeführt sein. Im Bereich eines Gerätegehäuses kann ein Sauerstoffzuschaltventil an die Vorrichtung zur Beatmung adaptiert werden. Es ist denkbar, das Atemgas zusätzlich mit Sauerstoff anzureichern, um die Patientenversorgung zu verbessern.

Das erfindungsgemäße Beatmungsgerät (20) ist so ausgelegt, dass es über einen Schlauch und ein Patienteninterface mit einem Patienten verbunden werden kann, um eine Beatmung bereitzustellen. Es umfasst eine Quelle für Atemgas (17), die beispielsweise als ein Elektromotor oder als Druckgasanschluss mit zumindest einem Ventil ausgebildet ist. Das Beatmungsgerät weist eine Einrichtung zur Ermittlung von Druck und/oder Fluss und/oder Volumen des Atemgases auf. Eine Steuereinheit (19) ist so ausgelegt, dass sie beispielsweise für einen Atemzyklus auf der Basis eines Vorgabewertes (25) und/oder auf Basis von Messwerten (24) für die Parameter Druck und/oder Flow und/oder Volumen einen Atemgasparameter bestimmt und die Quelle für Atemgas (17) derart reguliert, dass der Atemgasparameter appliziert wird. Die Steuereinheit kann die Parameter der Beatmung kontrolliert vorgeben und/oder zumindest teilweise assistiert oder adaptiv unter Berücksichtigung von Messwerten (24) oder Vorgabewerten (25). Die Steuereinheit (19) ist beispielsweise so ausgelegt, dass sie den aktuellen Druck und/oder Flow und/oder das Volumen von Atemgas bestimmt und den aktuellen Wert über das mit der Steuereinheit verbundene Bedien- und Informationssystem oder auf der Anzeige (3) darstellt.

Die Steuereinheit (19) ist außerdem so ausgelegt, dass sie bezogen auf einen oder mehrere Parameter Trendveränderungen ihrer Berechnungen über der Zeit bestimmt, wobei die Trendveränderungen auf der Anzeige angezeigt werden können. Das Beatmungsgerät weist zumindest einen Speicher (15) auf, der Teil der Steuereinheit (19) sein kann, in dem Messwerte (24) und/oder Anwendervorgaben (25) gespeichert werden. Das Beatmungsgerät weist beispielsweise auch zumindest eine Zeiteinheit (16) auf, die ein Teil der Steuereinheit (19) sein kann, die eine Zeitinformation bereitstellt oder abspeichert. Die Zeitinformation kann beispielsweise für die Einhaltung von Anwendervorgaben genutzt werden, um z.B. die Zeitdauer oder Druckrampen oder Inspirationsdauern vorzugeben und/oder einzuhalten. Weiterhin vergleicht die Steuereinheit (19) solche Parameter-Werte, die von einem Anwender vorgegeben wurden, beispielsweise obere und untere Druckgrenzen oder Druckrampen, oder ein Zielvolumen für die Ausatmung oder Einatmung, mit den aktuellen Werten und generiert eine Benutzer-Information zu Abweichungen von der Vorgabe. Die Benutzerinformation wird bevorzugt über das Bedien- und Informationssystem (3) graphisch visualisiert.

Die Steuereinheit (19) ist beispielsweise auch so ausgelegt, dass sie einen Druck oder ein Volumen oder einen Fluss zumindest zeitweise oder phasenweise bestimmt. Das Beatmungsgerät (20) weist dazu einen (pneumatischen oder elektronischen oder optischen) Drucksensor (14) und/oder einen Flusssensor (14) und oder einen Volumensensor (14) auf, der mit dem Patienteninterface (10) zumindest mittelbar gasführend verbunden ist.

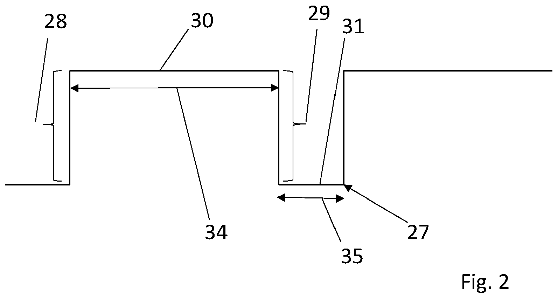

Figur 2 zeigt einen Druckverlauf der Beatmung. Das Beatmungsgerät (20) mit einer Atemgasquelle ist eingerichtet Atemgas für einen ersten Zeitraum (34, Tinsp) auf einem oberen Druckniveau (30, Pinsp) bereitzustellen und Atemgas für einen zweiten Zeitraum (35, Texsp) auf einem unteren Druckniveau (31, Pexsp) bereitzustellen. Die Sensoren (14) des Beatmungsgerätes ermitteln den Inspirationsfluss oder das -Volumen (28) beim Übergang von dem unteren Druckniveau (31) auf das obere Druckniveau (30) und den Exspirationsfluss oder das -Volumen (29) beim Übergang von dem oberen Druckniveau (30) auf das untere Druckniveau (31). Das Beatmungsgerät steuert eine Umschaltung von dem unteren Druckniveau (31) auf das obere Druckniveau (30) an, wenn der Fluss oder das Volumen auf dem unteren Druckniveau (31) oder im Übergang zu dem unteren Niveau (31) einen Triggerwert (27) erreicht hat, der einem Bruchteil der addierten Flüsse oder Volumina (28) und (29) entspricht. Der Bruchteil kann (28) + (29) / 2 sein.

Die Zeitdauer des zweiten Zeitraums (35, Texsp) dauert adaptiv so lange an, bis der Triggerwert (27) erreicht ist. Der Triggerwert (27) kann auch von einem Anwender vorgegeben sein.

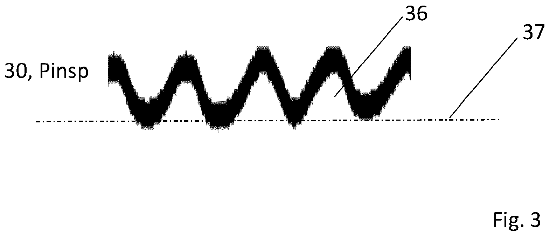

Figur 3 zeigt einen Volumenverlauf der Beatmung. Das Beatmungsgerät (20) gibt Atemgas für einen ersten Zeitraum (34, Tinsp) auf einem oberen Druckniveau (30, Pinsp) vor und Atemgas für einen zweiten Zeitraum (35, Texsp) auf einem unteren Druckniveau (31, Pexsp). Das Beatmungsgerät ermöglicht einem Patienten dabei eine Spontanatmung mit Inspirationen (29) und Exspirationen (28) zumindest auf dem oberen Druckniveau (30, Pinsp). Dabei ermittelt das Beatmungsgerät das Atemzugvolumen (36) sensorisch aus dem Fluss der Inspiration (29) und Exspiration (28). Das Atemzugvolumen (36) wird das Beatmungsgerät verwenden, zur Bestimmung eines Basisniveaus (37) des Atemvolumens. Das Basisniveau (37) ist dasjenige Volumen, welches der Patient in dem zweiten Zeitraum (35, Texsp) auf einem unteren Druckniveau (31, Pexsp) zumindest abatmen sollte. Das Basisniveau (37) kann auch zumindest teilweise oder vollständig von einem Anwender vorgegeben werden.

Erfindungsgemäß erfolgt eine Registrierung der Atemzugvolumina auf dem oberen Druckniveau und eine Ermittlung eines Basisniveaus. Diese Schwelle dient als Ausgangswert für die Exspiration.

Bei Auslösung einer Exspirationsphase, dem zweiten Zeitraum (35) mit dem unteren Druckniveau (31, Pexsp), wird ein Volumen unterhalb des Basisniveaus (37) abgegeben. Da dieses Volumen den intrapulmonalen Druck bestimmt, unter dem nicht weiter ausgeatmet werden darf, wird es als Bezugsvolumen für die Zeitdauer der Exspirationsphase verwendet. Atmet der Patient während der Exspirationsphase (35) ein, fällt das Volumen noch nicht ab und die Zeitdauer der Exspirationsphase (35, Texsp) bleibt so lange bestehen, bis das Bezugsvolumen erreicht wird.

Atmet der Patient zu Beginn der Exspirationsphase (35) aus, wird das Bezugsvolumen eher erreicht und die Exspirationsphase (35) wird früher beendet.

Damit ist es egal, in welcher Atemphase des Patienten die Exspirationsphase (35) einsetzt. Durch ein festgelegtes Bezugsvolumen passt sich die Zeitdauer der Exspirationsphase (35) dynamisch an.

Der Vorteil des Verfahrens besteht darin, dass zur Steuerung der Zeitdauer der Exspirationsphase (35) das stabile Volumensignal verwendet wird.

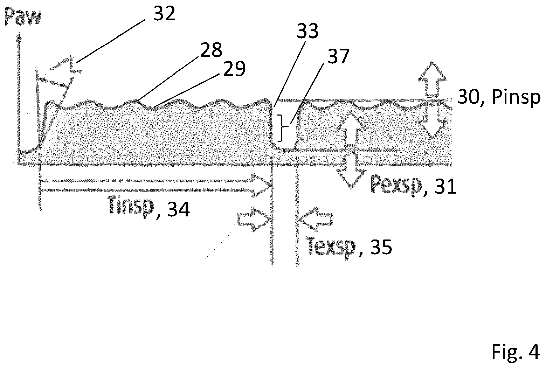

Figur 4 zeigt einen Druckverlauf der Beatmung. Das Beatmungsgerät (20) gibt Atemgas für einen ersten Zeitraum (34, Tinsp) auf einem oberen Druckniveau (30, Pinsp) vor und Atemgas für einen zweiten Zeitraum (35, Texsp) auf einem unteren Druckniveau (31, Pexsp). Das Beatmungsgerät ermöglicht einem Patienten dabei eine Spontanatmung mit Inspirationen (29) und Exspirationen (28) zumindest auf dem oberen Druckniveau (30, Pinsp). Dabei ermittelt das Beatmungsgerät das Atemzugvolumen (36) sensorisch aus dem Fluss der Inspiration (29) und Exspiration (28). Das Atemzugvolumen (36) wird das Beatmungsgerät verwenden, zur Bestimmung eines Basisniveaus (37) des Atemvolumens. Das Basisniveau (37) ist dasjenige Volumen, welches der Patient in dem zweiten Zeitraum (35, Texsp) auf einem unteren Druckniveau (31, Pexsp) zumindest abatmen sollte. Das Basisniveau (37) kann auch zumindest teilweise oder vollständig von einem Anwender vorgegeben werden. Das Beatmungsgerät senkt zur Einleitung des zweiten Zeitraums (35, Texsp) das Druckniveau ab, auf das untere Druckniveau (31, Pexsp). Dadurch kann der Patient erleichtert ausatmen. Das Beatmungsgerät erfasst das vom Patienten abgegebene Ausatemvolumen (38) und vergleicht das abgegebene Ausatemvolumen (38) mit dem Basisniveau (37). Erst wenn das Ausatemvolumen (38) dem Basisniveau (37) zumindest annähernd entspricht, schaltet das Beatmungsgerät wieder auf ein oberes Druckniveau (30, Pinsp) um und beendet somit den zweiten Zeitraum (35, Texsp) auf einem unteren Druckniveau (31, Pexsp).

Die Zeitdauer des zweiten Zeitraums (35, Texsp) dauert adaptiv so lange an, bis das Ausatemvolumen (38) den Wert des Basisniveaus (37) des Atemvolumens erreicht hat.

Das Beatmungsgerät kann die Absenkung des Druckniveaus von dem oberen (30) auf das untere Druckniveau (31, Pexsp) über eine adaptive oder einstellbare Rampe (33) steuern. Eine adaptive Rampe (33) berücksichtigt dabei beispielsweise die aktuellen Werte von Fluss oder Volumen des Patienten.

Das Beatmungsgerät kann den Anstieg des Druckniveaus von dem unteren (31) auf das obere Druckniveau (30) über eine adaptive oder einstellbare Rampe (32) steuern. Eine adaptive Rampe (32) berücksichtigt dabei beispielsweise die aktuellen Werte von Fluss oder Volumen des Patienten.

## Patentansprüche

1. Beatmungsgerät (20) mit einer Atemgasquelle (17) und einer Steuereinheit (19) die eingerichtet ist Atemgas für einen ersten, längeren Zeitraum (34, Tinsp) auf einem oberen Druckniveau (30, Pinsp) bereitzustellen und Atemgas für einen zweiten, kürzeren Zeitraum (35, Texsp) auf einem unteren Druckniveau (31, Pexsp) bereitzustellen, wobei das Beatmungsgerät Sensoren (14) aufweist, die Messwerte (24) des Atemgases (den Fluss und/oder Druck und/oder das Volumen des Atemgases) zumindest zeitweise erfassen wobei die Steuereinheit (19) den ersten, längeren Zeitraum (34, Tinsp) mit dem oberen Druckniveau (30, Pinsp) für zumindest zwei spontane Atemphasen des Patienten (Inspiration und eine Exspiration) bereitstellt bevor der zweite, kürzere Zeitraum (35, Texsp) auf einem unteren Druckniveau (31, Pexsp) vorgegeben wird (um eine vertiefte Exspiration zu ermöglichen), wobei Beatmungsgerät eine Spontanatmung eines Patienten auf dem oberen Druckniveau (30, Pinsp) für zumindest zwei aufeinanderfolgende Atemphasen ermöglicht und dabei ein Atemzugvolumen (36) sensorisch (14) erfasst , **dadurch gekennzeichnet, dass** die Steuereinheit ein Basisniveau (37) des Atemvolumens aus dem Messwert (24) des Atemzugvolumens (36) ermittelt, wobei die Steuereinheit (19) während des zweiten Zeitraums (35, Texsp) ein vom Patienten abgegebenes Ausatemvolumen (38) erfasst und die Zeitdauer des zweiten Zeitraums (35, Texsp) so lange andauert, bis das Ausatemvolumen (38) den Wert des Basisniveaus (37) im Wesentlichen erreicht hat und wobei das Ausatemvolumen (38) den Wert des Basisniveaus (37) nicht unterschreitet.

2. Beatmungsgerät (20) nach Anspruch 1 **dadurch gekennzeichnet, dass** das Beatmungsgerät Sensoren (14) aufweist, die Messwerte (24) des Atemgases (den Fluss und/oder Druck und/oder das Volumen des Atemgases) zumindest zeitweise erfassen wobei die Steuereinheit (19) einen Übergang zwischen den Druckniveaus (30, 31) und/oder den zweiten Zeitraum (35, Texsp) zumindest teilweise in Abhängigkeit von den Messwerten (24) steuert.

3. Beatmungsgerät (20) nach Anspruch 1 oder 2 **dadurch gekennzeichnet, dass** die Steuereinheit (19) den Übergang von dem unteren Druckniveau (31) auf das obere Druckniveau in Abhängigkeit von den Messwerten (24) für Fluss/Volumen steuert und der zweite Zeitraum (35, Texsp) insofern adaptiv ist.

4. Beatmungsgerät (20) nach zumindest einem der vorhergehenden Ansprüche 1 bis 3 **dadurch gekennzeichnet, dass** das Basisniveau (VT%) (37) von der Steuereinheit (19) aus einem Speicher (15) als Vorgabewert (25) entnommen wird.

5. Beatmungsgerät (20) nach zumindest einem der vorhergehenden Ansprüche **dadurch gekennzeichnet, dass** das Beatmungsgerät in einer Lernphase einen Inspirationsfluss (28) beim Übergang von dem unteren Druckniveau (31) auf das obere Druckniveau (30) erfasst und einen Exspirationsfluss (29) beim Übergang von dem oberen Druckniveau (30) auf das untere Druckniveau (31) erfasst wobei das Beatmungsgerät nachfolgend eine Umschaltung von dem unteren Druckniveau (31) auf das obere Druckniveau (30) ansteuert, wenn der Fluss auf dem unteren Druckniveau (31) einen Triggerwert (27) erreicht hat, der einem Bruchteil der addierten Flüsse (28) und (29) entspricht.

6. Beatmungsgerät (20) nach Anspruch 5 **dadurch gekennzeichnet, dass** der Triggerwert (27) (28) + (29) / 2 entspricht.

7. Beatmungsgerät (20) nach zumindest einem der vorhergehenden Ansprüche 5 und 6 **dadurch gekennzeichnet, dass** der Triggerwert (27) sensorisch bestimmt wird und/oder durch einen Anwender vorgegeben wird (Total Flow %).

8. Beatmungsgerät (20) nach zumindest einem der vorhergehenden Ansprüche mit einer Atemgasquelle die eingerichtet ist Atemgas für einen ersten Zeitraum (34, Tinsp) auf einem oberen Druckniveau (30, Pinsp) bereitzustellen und Atemgas für einen zweiten Zeitraum (35, Texsp) auf einem unteren Druckniveau (31, Pexsp) bereitzustellen **dadurch gekennzeichnet, dass** das Beatmungsgerät den Inspirationsfluss (28) beim Übergang von dem unteren Druckniveau (31) auf das obere Druckniveau (30) erfasst und das Beatmungsgerät den Exspirationsfluss (29) beim Übergang von dem oberen Druckniveau (30) auf das untere Druckniveau (31) erfasst wobei das Beatmungsgerät eine Umschaltung von dem unteren Druckniveau (31) auf das obere Druckniveau (30) ansteuert, wenn der Fluss auf dem unteren Druckniveau (31) einen Triggerwert (27) erreicht hat, der einem Bruchteil der addierten Flüsse (28) und (29) entspricht.

9. Beatmungsgerät (20) nach zumindest einem der vorhergehenden Ansprüche **dadurch gekennzeichnet, dass** eine Zeitdauer des zweiten Zeitraums (35, Texsp) in dem Bereich 0,4 bis 1,8 sec, bevorzugt 0,5 - 3,0 sec liegt, in Abhängigkeit der Schwere der Oxygenierungsstörung.

10. Beatmungsgerät (20) nach zumindest einem der vorhergehenden Ansprüche **dadurch gekennzeichnet, dass** eine Anzahl der Phasen auf dem unteren Druckniveau (31, Pexsp) 5 - 15 pro Min, bevorzugt 7 - 12 pro Min entspricht, abhängig vom Ausmaß der gewünschten Unterstützung der Ventilation und der Elimination von CO2.

11. Beatmungsgerät (20) nach zumindest einem der vorhergehenden Ansprüche **dadurch gekennzeichnet, dass** ein Atemzeitverhältnis von Zeitraum (34, Tinsp) zu Zeitraum (35, Texsp) größer 3 zu 1 ist, bevorzugt größer 4 zu 1 ist.

## Claims

1. A breathing apparatus (20) having a breathing gas source (17) and a control unit (19) which is adapted to provide breathing gas for a first, longer period of time (34, Tinsp) at an upper pressure level (30, Pinsp) and to provide breathing gas for a second, shorter period of time (35, Texsp) at a lower pressure level (31, Pexsp), wherein the breathing apparatus has sensors (14) which detect measured values (24) of the breathing gas (the flow and/or pressure and/or the volume of the breathing gas) at least intermittently, wherein the control unit (19) provides the first, longer period of time (34, Tinsp) with the upper pressure level (30, Pinsp) for at least two spontaneous breathing phases of the patient (inspiration and an expiration) before the second, shorter period of time (35, Texsp) at a lower pressure level (31, Pexsp) is specified (in order to make possible a deepened expiration), wherein the breathing apparatus makes possible a spontaneous respiration of a patient at the upper pressure level (30, Pinsp) for at least two consecutive breathing phases and detects a tidal volume (36) by sensor technology (14), **characterized in that** the control unit establishes a base level (37) of the respiratory volume from the measured value (24) of the tidal volume (36), wherein the control unit (19) detects an expiratory volume (38) exhaled by the patient during the second period of time (35, Texsp) and the length of the second period of time (35, Texsp) lasts until such time as the expiratory volume (38) has substantially reached the value of the base level (37) and wherein the expiratory volume (38) does not fall below the value of the base level (37).

2. The breathing apparatus (20) according to Claim 1, **characterized in that** the breathing apparatus has sensors (14) which detect measured values (24) of the breathing gas (the flow and/or pressure and/or the volume of the breathing gas) at least intermittently, wherein the control unit (19) controls a transition between the pressure levels (30, 31) and/or the second period of time (35, Texsp) at least partially as a function of the measured values (24).

3. The breathing apparatus (20) according to Claim 1 or 2, **characterized in that** the control unit (19) controls the transition from the lower pressure level (31) to the upper pressure level as a function of the measured values (24) for flow/volume and the second period of time (35, Texsp) is adaptive in this respect.

4. The breathing apparatus (20) according to at least one of the preceding Claims 1 to 4, **characterized in that** the base level (VT%) (37) is extracted as a set value (25) by the control unit (19) from a memory (15).

5. The breathing apparatus (20) according to at least one of the preceding claims, **characterized in that**, in a learning phase, the breathing apparatus detects an inspiration flow (28) during the transition from the lower pressure level (31) to the upper pressure level (30) and detects an expiration flow (29) during the transition from the upper pressure level (30) to the lower pressure level (31), wherein the breathing apparatus subsequently actuates a switchover from the lower pressure level (31) to the upper pressure level (30) when the flow at the lower pressure level (31) has reached a trigger value (27) which corresponds to a fraction of the added flows (28) and (29).

6. The breathing apparatus (20) according to Claim 5, **characterized in that** the trigger value (27) corresponds to (28) + (29) / 2.

7. The breathing apparatus (20) according to at least one of the preceding Claims 5 and 6, **characterized in that** the trigger value (27) is determined by sensor technology and/or is specified by a user (total flow %).

8. The breathing apparatus (20) according to at least one of the preceding claims, having a breathing gas source which is adapted to provide breathing gas for a first period of time (34, Tinsp) at an upper pressure level (30, Pinsp) and to provide breathing gas for a second period of time (35, Texsp) at a lower pressure level (31, Pexsp), **characterized in that** the breathing apparatus detects the inspiration flow (28) during the transition from the lower pressure level (31) to the upper pressure level (30) and the breathing apparatus detects the expiration flow (29) during the transition from the upper pressure level (30) to the lower pressure level (31), wherein the breathing apparatus actuates a switchover from the lower pressure level (31) to the upper pressure level (30) when the flow at the lower pressure level (31) has reached a trigger value (27) which corresponds to a fraction of the added flows (28) and (29).

9. The breathing apparatus (20) according to at least one of the preceding claims, **characterized in that** a length of the second period of time (35, Texsp) lies in the region of 0.4 to 1.8 sec, preferably 0.5 - 3.0 sec, as a function of the severity of the oxygenation disturbance.

10. The breathing apparatus (20) according to at least one of the preceding claims, **characterized in that** a number of the phases at the lower pressure level (31, Pexsp) corresponds to 5 - 15 per min, preferably 7 - 12 per min, depending on the extent of the desired support of the ventilation and of the elimination of CO2.

11. The breathing apparatus (20) according to at least one of the preceding claims, **characterized in that** an inspiration-to-expiration ratio from period of time (34, Tinsp) to period of time (35, Texsp) is greater than 3 to 1, is preferably greater than 4 to 1.

## Revendications

1. Appareil respiratoire (20) doté d'une source de gaz respiratoire (17) et d'une unité de commande (19) conçue pour fournir du gaz respiratoire pour une première période plus longue (34, Tinsp) à un niveau de pression supérieur (30, Pinsp) et pour fournir du gaz respiratoire pour une deuxième période plus courte (35, Texsp) à un niveau de pression inférieur (31, Pexsp), dans lequel l'appareil respiratoire présente des capteurs (14) détectant au moins temporairement des valeurs de mesure (24) du gaz respiratoire (le flux et/ou la pression et/ou le volume du gaz respiratoire), dans lequel l'unité de commande (19) fournit la première période plus longue (34, Tinsp) avec le niveau de pression supérieur (30, Pinsp) pour au moins deux phases respiratoires spontanées du patient .(inspiration et une expiration) avant la prescription de la deuxième période plus courte (35, Texsp) à un niveau de pression inférieur (31, Pexsp) (afin de permettre une expiration approfondie), dans lequel l'appareil respiratoire permet une respiration spontanée d'un patient au niveau de pression supérieur (30, Pinsp) pour au moins deux phases respiratoires consécutives, tout en détectant un volume d'inspiration (36) à l'aide des capteurs (14), **caractérisé en ce que** l'unité de commande détermine un niveau de base (37) du volume respiratoire à partir de la valeur de mesure (24) du volume d'inspiration (36), dans lequel, pendant la deuxième période plus courte (35, Texsp), l'unité de commande (19) détecte un volume d'expiration (38) délivré par le patient et la durée de la deuxième période plus courte (35, Texsp) se prolonge jusqu'à ce que le volume d'expiration (38) ait quasiment atteint la valeur du niveau de base (37) et dans lequel le volume d'expiration (38) ne dépasse pas la valeur du niveau de base (37).

2. Appareil respiratoire (20) selon la revendication 1, **caractérisé en ce que** l'appareil respiratoire présente des capteurs (14) détectant au moins temporairement des valeurs de mesure (24) du gaz respiratoire (le flux et/ou la pression et/ou le volume du gaz respiratoire), dans lequel l'unité de commande (19) commande une transition entre les niveaux de pression (30, 31) et/ou la deuxième période (35, Texsp) au moins partiellement en fonction des valeurs de mesure (24).

3. Appareil respiratoire (20) selon la revendication 1 ou 2, **caractérisé en ce que** l'unité de commande (19) commande la transition du niveau de pression inférieur (31) au niveau de pression supérieur en fonction des valeurs de mesure (24) pour le flux/le volume et la deuxième période (35, Texsp) est par conséquent adaptative.

4. Appareil respiratoire (20) selon l'une au moins des revendications précédentes 1 à 4, **caractérisé en ce que** le niveau de base (VT%) (37) est prélevé par l'unité de commande (19) dans une mémoire (15) en tant que valeur de consigne (25).

5. Appareil respiratoire (20) selon l'une au moins des revendications précédentes, **caractérisé en ce qu'**au cours d'une phase d'apprentissage, l'appareil respiratoire détecte un flux d'inspiration (28) lors de la transition du niveau de pression inférieur (31) au niveau de pression supérieur (30) et détecte un flux d'expiration (29) lors de la transition du niveau de pression supérieur (30) au niveau de pression inférieur (31), dans lequel l'apparéil respiratoire actionne ensuite un passage du niveau de pression inférieur (31) au niveau de pression supérieur (30), lorsque le flux a atteint une valeur de déclenchement (27) au niveau de pression inférieur (31), laquelle correspond à une fraction de la somme des flux (28) et (29).

6. Appareil respiratoire (20) selon la revendication 5, **caractérisé en ce que** la valeur de déclenchement (27) correspond à (28) + (29) / 2.

7. Appareil respiratoire (20) selon l'une au moins des revendications précédentes 5 et 6, **caractérisé en ce que** la valeur de déclenchement (27) est déterminée par capteur et/ou prédéfinie par un utilisateur (flux total %).

8. Appareil respiratoire (20) selon l'une au moins des revendications précédentes, doté d'une source de gaz respiratoire conçue pour fournir du gaz respiratoire pour une première période (34, Tinsp) à un niveau de pression supérieur (30, Pinsp) et pour fournir du gaz respiratoire pour une deuxième période (35, Texsp) à un niveau de pression inférieur (31, Pexsp), **caractérisé en ce que** l'appareil respiratoire détecte le flux d'inspiration (28) lors de la transition du niveau de pression inférieur (31) au niveau de pression supérieur (30) et l'appareil respiratoire détecte le flux d'expiration (29) lors de la transition du niveau de pression supérieur (30) au niveau de pression inférieur (31), dans lequel l'appareil respiratoire actionne un passage du niveau de pression inférieur (31) au niveau de pression supérieur (30), lorsque le flux a atteint une valeur de déclenchement (27) au niveau de pression inférieur (31), laquelle correspond à une fraction de la somme des flux (28) et (29).

9. Appareil respiratoire (20) selon l'une au moins des revendications précédentes, **caractérisé en ce qu'**une durée de la deuxième période (35, Texsp) se situe dans la plage de 0,4 à 1,8 secondes, de préférence de 0,5 à 3,0 secondes, en fonction de la gravité du défaut d'oxygénation.

10. Appareil respiratoire (20) selon l'une au moins des revendications précédentes, **caractérisé en ce qu'**un nombre des phases au niveau de pression inférieur (31, Pexsp) est compris-entre 5 et 15 par minute, de préférence entre 7 et 12 par minute, en fonction du degré d'aide à la ventilation souhaité et de l'élimination de CO2.

11. Appareil respiratoire (20) selon l'une au moins des revendications précédentes, **caractérisé en ce qu'**un rapport de temps de respiration entre la période (34, Tinsp) et la période (35, Texsp) est supérieur à 3 à 1, de préférence supérieur à 4 à 1.
